Europäisches Patentamt

**European Patent Office**

**Office européen des brevets**

(11) Publication number: **0 411 684 A2**

## EUROPEAN PATENT APPLICATION

(21) Application number: **90201852.2**

(22) Date of filing: **10.07.90**

(51) Int. Cl.⁵: **C12N 15/50**, C07K 15/00, A61K 39/215

(30) Priority: **12.07.89 NL 8901794**

(43) Date of publication of application:
**06.02.91 Bulletin 91/06**

(84) Designated Contracting States:
**AT BE CH DE DK ES FR GB GR IT LI LU NL SE**

(71) Applicant: **DUPHAR INTERNATIONAL RESEARCH B.V**
**C.J. van Houtenlaan 36**
**NL-1381 CP Weesp(NL)**

(72) Inventor: **Vennema, Harry**
**c/o INT. OCTROOIBUREAU Zoan B.V, P.O. Box 140**
**NL-1380 AC Weesp(NL)**
Inventor: **Rottier, Peter J.**
**c/o INT. OCTROOIBUREAU Zoan B.V., P.O. Box 140**
**NL-1380 Weesp(NL)**

(74) Representative: **Muis, Maarten et al**
**OCTROOIBUREAU ZOAN B.V. P.O. Box 140**
**NL-1380 AC Weesp(NL)**

(54) **New antigenically active proteins/peptides, and feline infectious virus (FIPV)-vaccines.**

(57) The invention relates to two new antigenic proteins/peptides which can be used to stimulate the immunity of cats (Felidae) against feline infectious peritonitis virus (FIPV), i.e. the M-protein and the N-protein. The invention also relates to vaccines which comprise such antigenic proteins or peptides.

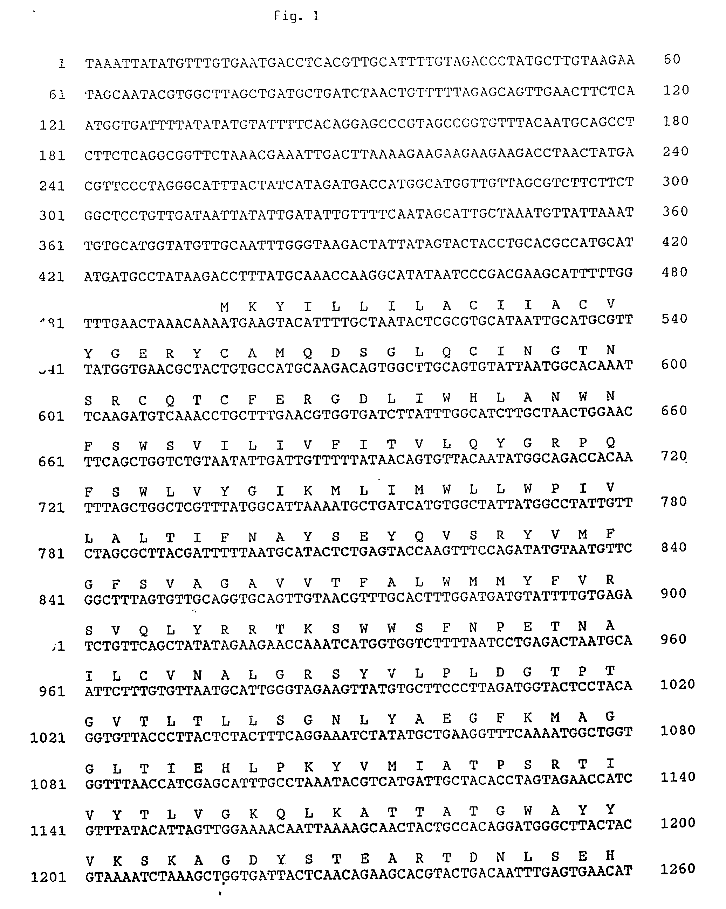

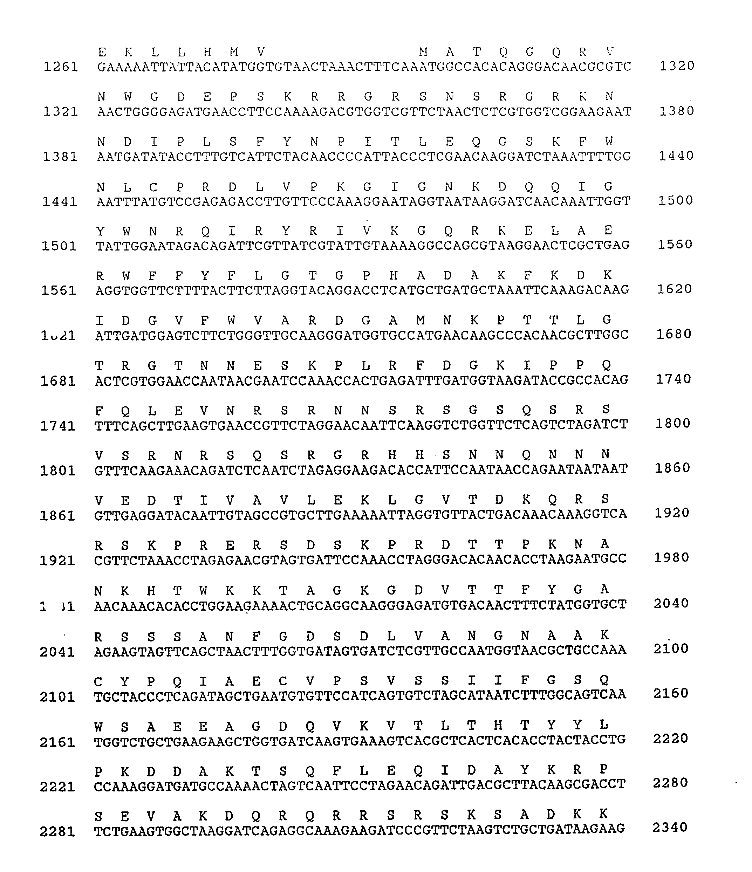

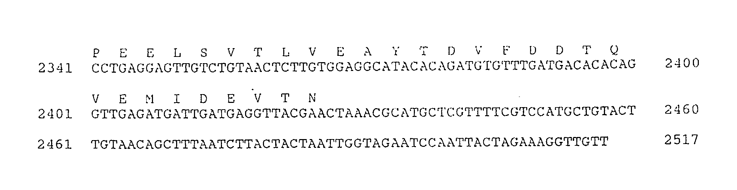

The nucleotide sequence and amino acid sequence of the M-protein and the N-protein are indicated in figure 1.

EP 0 411 684 A2

# NEW ANTIGENICALLY ACTIVE PROTEINS/PEPTIDES, AND FELINE INFECTIOUS PERITONITIS VIRUS (FIPV)-VACCINES

The present invention relates to the cloning and the determination of the base sequence of the genes for two structrual proteins of FIPV, which have proved to be capable of producing an immune response in cats: the M-protein and the N-protein.

The invention also relates to the amino acid sequence of the M-protein and the N-protein (which can be directly derived from the nucleic acid sequence), and hence to the proteins and the fragments thereof. The invention further relates to vaccines which comprise one or both proteins and/or derivatives of one or both proteins.

Feline Infectious Peritonitis is an infectious disease, which usually is lethal. The disease is caused by the Feline Infectious Peritonitis virus (FIPV). The clinical symptoms of a naturally acquired FIPV infection are not very characteristic initially. The cats are anaemic, show anorexia, total malaise, and fever. In a later stage of the disease a perceptible swelling of the belly often occurs. Said swelling is caused by the accumulation of ascites liquid, in quantities of a few millilitres to one litre. Upon necropsy a wide-spread peritonitis is always found, while the organs in the abdomen are often covered with a thick fibrinous exsudate. Lesions in the form of greyish-white swellings are observed in liver, lung, spleen, omentum, intestines and kidneys.

Humoral antibodies against FIPV do not provide any protection against an FIPV infection. On the other hand, a cat which has developed antibodies against FIPV as a result of an earlier infection, will develop clinical phenomena and lesions much sooner and will survive much shorter (the "early death" syndrome) than a cat with a first infection. The presumable explanation of this phenomenon is the so-called antibody-dependent enhancement (ADE), a phenomenon which has also been observed in herpes viridae, pox viridae, rhabdo viridae, flavi viridae, alpha viridae, reo viridae and bunya viridae. The phenomenon is probably based on binding of virus antibody complexes to the Fc-receptors of macrophages. This binding is said to be more efficient than binding between macrophages and virus without the intermediary of antibodies. The result then is that infection occurs more rapidly and more efficient than when the virus binds in a non-complexed form.

An active vaccine against FIPV has so far not been found and therapeutic measures have proved to be ineffective. Prophylactic measures cannot be taken either, since there is insufficient knowledge about pathogenesis and the way in which the virus is transferred.

Recently cats were infected with a vaccinia virus live recombinant carrier, in which the Spike protein of FIVP is cloned. All infected cats showed "early death" caused by ADE, as if they had been infected with intact live FIPV. This has demonstrated that the Spike protein of FIVP plays a role in the occurrence of ADE.

The Feline Infectious Peritonitis virus belongs to the Corona viridae. Corona viruses are spherical particles having a diameter of approximately 100 nm. They consist of a spiral-like nucleocapsid which is enveloped by a lipide-containing envelope. There are three structural proteins: the nucleocapsid protein (N: 40-50K), and two membrane proteins; the Spike protein (S: 180-20K) and the matrix protein (M: 25-30K). The viruses replicate in the cytoplasma where the relevant gene(s) of the approximately 30 kb RNA-genome is (are) translated to RNA-polymerase(s). A full-length negative strand is then synthetised which then serves as a template for the synthesis of new genome-RNA and 5-7 subgenomic messenger-RNAs. These m-RNAs have a "cap" structure, are polyadenylated, and form a $3'$- "nested set". This means that the m-RNAs have equal $3'$-ends but a different $5'$-length.

The proteins to which the invention relates each comprise at least one region which is immunogenic. Such proteins or fragments thereof may theoretically be used as components for a vaccine against FIPV. When fragments are used instead of the whole proteins, they may be used coupled to known carriers as KLH or BSA.

The peptide fragments or proteins according to the invention which comprise at least one antigenically active pep tide fragment may be prepared according to methods known for the preparation of peptides and proteins.

First of all, the peptides may be prepared synthetically by means of known techniques starting from the individual amino acids or smaller peptide fragments.

The peptides and proteins may also be obtained biosynthetically while using recombinant DNA techniques and expression systems, for example, by:

.a) transformation of host cells with an expression vector which comprises a DNA, coding for an (the) antigenic determinant(s) (peptides in general);

b) expressing the genome inserted in the expression vector;

c) harvesting the cell culture, and

d) isolating the synthetised peptide (protein).

The invention therefore also relates to a method of preparing a DNA molecule which codes for proteins or pep tides according to the invention. Such a method comprises the following steps:

a) isolating single-stranded RNA of FIP;

b) synthesis of a cDNA strand which is complementary to the RNA strand mentioned in a);

c) synthesis of a second cDNA strand, using the first cDNA strand as a template. The double-stranded DNA obtained in this manner may be inserted in a manner known per se into an expression vector as a result of which a recombinant expression vector is formed. The vector may be brought into a suitable host cell, for example, by transformation.

The proteins or pep tides may also be expressed in the target animal in vivo . For that purpose, the genes which code for the N-protein and/or the M-protein or parts thereof may be incorporated in live recombinant carriers (LRC), for example,:

1) Vaccinia-LRC
2) Herpes-LRC
3) Adeno-LRC
4) Adeno-associated-LRC
5) Sindbis-LRC
6) Corona-LRC
7) Bacterial-LRC

Administration of the LRC so constructed to the target animal leads to expression of the inserted genes in all infected cells (besides expression of carrier proteins).

The invention will now be described in more detail with reference to the ensuing specific example.

## EXAMPLE

### Virus and tissue culture

FIPV strain 79-1146 was purified twice by means of end-point dilution on fcwf-D cells and was then used for making virus stocks.

### a) Isolation of viral genome RNA

Fcwf-D cells were infected with FIPV 79-1146 with a multiplicity of infection of 0.1 $TCID_{50}$. Sixteen hours after infection the medium was harvested and centrifuged at 1000xg for 10 minutes so as to remove cell debris. NaCl and polyethylene glycol (BDH, Poole, England) were added to a final concentration of 2.33% (wt/vol) and 10% (wt/vol), respectively. The mixture was centrifuged at 4°C and 10,000x g for 16 hours. The pellet was resuspended in TESV buffer (0.02 M Tris-Cl pH 7.4, 1mm EDTA, 0.1 M NaCl). After centrifuging at 10,000x g for 10 minutes the virus suspension was brought on a 10% (wt/vol) sucrose cushion which was placed on top of a 20-50% (wt/vol) sucrose gradient (sucrose solutions in TESV). The gradient was centrifuged at 16,000 rpm at 4°C for 16 hours in an SW 27.0 rotor. The virus band was harvested and diluted three times in TESV. The virus was pelleted by centrifuging at 4°C and 250,000 xg for 90 minutes and then incubated at 37°C for 30 minutes in TESV, 0.5% SDS, 0.5 mg/ml of proteinase K. Finally, the RNA was extracted with phenol-chloroform, followed by ether extraction and ethanol precipitation. In this manner approximately 5 ug of genome RNA could be obtained from 2 x $10^8$ infected cells.

### b) Cloning of FIPV genome RNA

The synthesis of cDNA was carried out as described by Gubler and Hoffman (Gene 25 (1983), 263-269). Calf thymus DNA pentamers as ('random') primers and FIPV genome RNA denaturated with methyl mercury hydroxide were used. The double-stranded cDNA was tailed with dC-residues and then ligated in PstI digerated, dG-tailed pUC9. The recombinant plasmids thus obtained were used for transformation of E.coli strain JM109 as described by hanahan (J.Mol.Biol. 166 (1983), 557-580). In this manner a cDNA bank of 400 transformants was obtained. The plasmids comprise inserts having a length up to 6 kb with an average size of 2.5 kb.

### Selection of N/M sequences-comprising recombinants

Recombinants with N/M-specific sequences could be indentified by using the fact that said sequences are situated at the 3' end of the genome (J .Gen.Virol. 68 (1987), 995-1002). Southern blots of recombinant plasmids were hybridised with $^{32}P$ labelled RNA fractions which were enriched for the 1.4 kb FIPV RNA. The said fraction was obtained by isokinetic sucrose gradient centrifugation of total poly(A) + RNA from FIPV infected cells. The plasmids pUC-FIPV-B12, C12 and E7 hybridised with this probe and were therefore used for further analysis.

### d) Characterisation of cloned sequences

cDNA clone B12 after hybridisation and restriction enzyme analysis proved to comprise the M-gene and N-gene and was used for sequence analysis. Restriction fragments were separated on agarose gels, isolated by means of NA45-membranes (Schleicher and Schuell) and subcloned in M13 vectors. Sequence analysis was carried out according to the dideoxy method (Sanger et al, Proc.Natl.Acad.Sci. USA 74 (1977), 5463-5467). Both the universal M13 primer and internal synthetic primers (17-mer) were used.

The data were analysed by means of a DEC 20/60 computer and the programmes of Staden, Nucl.Acid.Res. 10 (1982), 4731-4751).

e) Nucleotide sequences

The nucleotide sequence of the M-gene is shown in Figure 1. The coding sequence of the M-gene begins with an ATG-codon in positions 496-498, and ends with the stop codon TAA in positions 1282-1284. The coding part of the M-gene thus comprises 786 nuleic acids and codes for a protein of 262 amino acids.

The coding sequence of the N-gene begins with an ATG codon in positions 1297-1299 and ends with the stop codon TAA in positions 2428-2430. The coding part of the N-gene thus comprises 1131 nucleic acids and codes for a protein of 377 amino acids.

**Claims**

1. Genes which code for the M-protein or the N-protein of feline infectious peritonitis virus (FIPV), characterised by the nucleotide sequence as shown in Figure 1.

2. A protein, characterised by the amino acid sequence as shown in Figure 1, or a part thereof.

3. An immunogen, characterised in that it comprises a protein or a peptide as claimed in Claim 2, whether or not coupled to a carrier.

4. A vaccine, characterised in that it comprises an immunogenic protein or peptide as claimed in Claim 3,.

5. A method of preparing a protein or peptide as claimed in Claim 2, characterised in that

a) the protein or peptide is prepared synthetically in a manner known per se from individual amino acids and/or by coupling smaller peptides; or

b) a host cell is transformed with an expression vector which comprises a DNA coding for a protein or peptide as claimed in Claim 2, and that the genome inserted in the expression vector is expressed, or

c) in vivo , cells are infected in the target animal with an LRC which comprises DNA which codes for a protein or peptide as claimed in Claim 2, and that protein or peptide is expressed in the said host cells.

6. A DNA molecule which comprises a nucleotide sequence wich codes for a protein or peptide as claimed in Claim 2, or which codes for a polypeptide which comprises a protein or peptide as claimed in Claim 2.

7. A recombinant DNA expression vector which expresses the whole protein or peptide or a part thereof as claimed in Claim 2, comprising an operon with initiator sequences and terminator sequences and a nucleotide sequence which codes for the said protein or peptide, the said nucleotide sequence being between the initiator sequences and the terminator sequences of the operon.

8. A Live Recombinant Carrier which comprises an expression vector as defined in Claim 7.

9. A host cell which comprises a recombinant DNA expression vector and/or a DNA molecule as claimed in Claim 6,7 or 8.

10. A method of preparing a DNA molecule which codes for a protein or peptide as claimed in Claim 2, characterised in that

a) single-stranded FIPV-RNA is isolated;

b) a cDNA strand complementary to the said RNA strand is synthetised;

c) a second strand cDNA is synthetised with the first cDNA strand as a template.

Fig. 1

```
   1  TAAATTATATGTTTGTGAATGACCTCACGTTGCATTTTGTAGACCCTATGCTTGTAAGAA   60

  61  TAGCAATACGTGGCTTAGCTGATGCTGATCTAACTGTTTTTAGAGCAGTTGAACTTCTCA  120

 121  ATGGTGATTTTATATATGTATTTTCACAGGAGCCCGTAGCCGGTGTTTACAATGCAGCCT  180

 181  CTTCTCAGGCGGTTCTAAACGAAATTGACTTAAAAGAAGAAGAAGAAGACCTAACTATGA  240

 241  CGTTCCCTAGGGCATTTACTATCATAGATGACCATGGCATGGTTGTTAGCGTCTTCTTCT  300

 301  GGCTCCTGTTGATAATTATATTGATATTGTTTTCAATAGCATTGCTAAATGTTATTAAAT  360

 361  TGTGCATGGTATGTTGCAATTTGGGTAAGACTATTATAGTACTACCTGCACGCCATGCAT  420

 421  ATGATGCCTATAAGACCTTTATGCAAACCAAGGCATATAATCCCGACGAAGCATTTTTGG  480

                  M  K  Y  I  L  L  I  L  A  C  I  I  A  C  V
 481  TTTGAACTAAACAAAATGAAGTACATTTTGCTAATACTCGCGTGCATAATTGCATGCGTT  540

         Y  G  E  R  Y  C  A  M  Q  D  S  G  L  Q  C  I  N  G  T  N
 541  TATGGTGAACGCTACTGTGCCATGCAAGACAGTGGCTTGCAGTGTATTAATGGCACAAAT  600

         S  R  C  Q  T  C  F  E  R  G  D  L  I  W  H  L  A  N  W  N
 601  TCAAGATGTCAAACCTGCTTTGAACGTGGTGATCTTATTTGGCATCTTGCTAACTGGAAC  660

         F  S  W  S  V  I  L  I  V  F  I  T  V  L  Q  Y  G  R  P  Q
 661  TTCAGCTGGTCTGTAATATTGATTGTTTTTATAACAGTGTTACAATATGGCAGACCACAA  720

         F  S  W  L  V  Y  G  I  K  M  L  I  M  W  L  L  W  P  I  V
 721  TTTAGCTGGCTCGTTTATGGCATTAAAATGCTGATCATGTGGCTATTATGGCCTATTGTT  780

         L  A  L  T  I  F  N  A  Y  S  E  Y  Q  V  S  R  Y  V  M  F
 781  CTAGCGCTTACGATTTTTAATGCATACTCTGAGTACCAAGTTTCCAGATATGTAATGTTC  840

         G  F  S  V  A  G  A  V  V  T  F  A  L  W  M  M  Y  F  V  R
 841  GGCTTTAGTGTTGCAGGTGCAGTTGTAACGTTTGCACTTTGGATGATGTATTTTGTGAGA  900

         S  V  Q  L  Y  R  R  T  K  S  W  W  S  F  N  P  E  T  N  A
 901  TCTGTTCAGCTATATAGAAGAACCAAATCATGGTGGTCTTTTAATCCTGAGACTAATGCA  960

         I  L  C  V  N  A  L  G  R  S  Y  V  L  P  L  D  G  T  P  T
 961  ATTCTTTGTGTTAATGCATTGGGTAGAAGTTATGTGCTTCCCTTAGATGGTACTCCTACA 1020

         G  V  T  L  T  L  L  S  G  N  L  Y  A  E  G  F  K  M  A  G
1021  GGTGTTACCCTTACTCTACTTTCAGGAAATCTATATGCTGAAGGTTTCAAAATGGCTGGT 1080

         G  L  T  I  E  H  L  P  K  Y  V  M  I  A  T  P  S  R  T  I
1081  GGTTTAACCATCGAGCATTTGCCTAAATACGTCATGATTGCTACACCTAGTAGAACCATC 1140

         V  Y  T  L  V  G  K  Q  L  K  A  T  T  A  T  G  W  A  Y  Y
1141  GTTTATACATTAGTTGGAAAACAATTAAAAGCAACTACTGCCACAGGATGGGCTTACTAC 1200

         V  K  S  K  A  G  D  Y  S  T  E  A  R  T  D  N  L  S  E  H
1201  GTAAAATCTAAAGCTGGTGATTACTCAACAGAAGCACGTACTGACAATTTGAGTGAACAT 1260
```

```
        E  K  L  L  H  M  V                             M  A  T  Q  G  Q  R  V
1261    GAAAAATTATTACATATGGTGTAACTAAACTTTCAAATGGCCACACAGGGACAACGCGTC      1320

        N  W  G  D  E  P  S  K  R  R  G  R  S  N  S  R  G  R  K  N
1321    AACTGGGGAGATGAACCTTCCAAAAGACGTGGTCGTTCTAACTCTCGTGGTCGGAAGAAT      1380

        N  D  I  P  L  S  F  Y  N  P  I  T  L  E  Q  G  S  K  F  W
1381    AATGATATACCTTTGTCATTCTACAACCCCATTACCCTCGAACAAGGATCTAAATTTTGG      1440

        N  L  C  P  R  D  L  V  P  K  G  I  G  N  K  D  Q  Q  I  G
1441    AATTTATGTCCGAGAGACCTTGTTCCCAAAGGAATAGGTAATAAGGATCAACAAATTGGT      1500

        Y  W  N  R  Q  I  R  Y  R  I  V  K  G  Q  R  K  E  L  A  E
1501    TATTGGAATAGACAGATTCGTTATCGTATTGTAAAAGGCCAGCGTAAGGAACTCGCTGAG      1560

        R  W  F  F  Y  F  L  G  T  G  P  H  A  D  A  K  F  K  D  K
1561    AGGTGGTTCTTTTACTTCTTAGGTACAGGACCTCATGCTGATGCTAAATTCAAAGACAAG      1620

        I  D  G  V  F  W  V  A  R  D  G  A  M  N  K  P  T  T  L  G
1621    ATTGATGGAGTCTTCTGGGTTGCAAGGGATGGTGCCATGAACAAGCCCACAACGCTTGGC      1680

        T  R  G  T  N  N  E  S  K  P  L  R  F  D  G  K  I  P  P  Q
1681    ACTCGTGGAACCAATAACGAATCCAAACCACTGAGATTTGATGGTAAGATACCGCCACAG      1740

        F  Q  L  E  V  N  R  S  R  N  N  S  R  S  G  S  Q  S  R  S
1741    TTTCAGCTTGAAGTGAACCGTTCTAGGAACAATTCAAGGTCTGGTTCTCAGTCTAGATCT      1800

        V  S  R  N  R  S  Q  S  R  G  R  H  H  S  N  N  Q  N  N
1801    GTTTCAAGAAACAGATCTCAATCTAGAGGAAGACACCATTCCAATAACCAGAATAATAAT      1860

        V  E  D  T  I  V  A  V  L  E  K  L  G  V  T  D  K  Q  R  S
1861    GTTGAGGATACAATTGTAGCCGTGCTTGAAAAATTAGGTGTTACTGACAAACAAAGGTCA      1920

        R  S  K  P  R  E  R  S  D  S  K  P  R  D  T  T  P  K  N  A
1921    CGTTCTAAACCTAGAGAACGTAGTGATTCCAAACCTAGGGACACAACACCTAAGAATGCC      1980

        N  K  H  T  W  K  K  T  A  G  K  G  D  V  T  T  F  Y  G  A
1981    AACAAACACACCTGGAAGAAAACTGCAGGCAAGGGAGATGTGACAACTTTCTATGGTGCT      2040

        R  S  S  S  A  N  F  G  D  S  D  L  V  A  N  G  N  A  A  K
2041    AGAAGTAGTTCAGCTAACTTTGGTGATAGTGATCTCGTTGCCAATGGTAACGCTGCCAAA      2100

        C  Y  P  Q  I  A  E  C  V  P  S  V  S  S  I  I  F  G  S  Q
2101    TGCTACCCTCAGATAGCTGAATGTGTTCCATCAGTGTCTAGCATAATCTTTGGCAGTCAA      2160

        W  S  A  E  E  A  G  D  Q  V  K  V  T  L  T  H  T  Y  Y  L
2161    TGGTCTGCTGAAGAAGCTGGTGATCAAGTGAAAGTCACGCTCACTCACACCTACTACCTG      2220

        P  K  D  D  A  K  T  S  Q  F  L  E  Q  I  D  A  Y  K  R  P
2221    CCAAAGGATGATGCCAAAACTAGTCAATTCCTAGAACAGATTGACGCTTACAAGCGACCT      2280

        S  E  V  A  K  D  Q  R  Q  R  R  S  R  S  K  S  A  D  K  K
2281    TCTGAAGTGGCTAAGGATCAGAGGCAAAGAAGATCCCGTTCTAAGTCTGCTGATAAGAAG      2340
```

6

```
      P   E   E   L   S   V   T   L   V   E   A   Y   T   D   V   F   D   D   T   Q
2341  CCTGAGGAGTTGTCTGTAACTCTTGTGGAGGCATACACAGATGTGTTTGATGACACACAG        2400

      V   E   M   I   D   E   V   T   N
2401  GTTGAGATGATTGATGAGGTTACGAACTAAACGCATGCTCGTTTTCGTCCATGCTGTACT        2460

2461  TGTAACAGCTTTAATCTTACTACTAATTGGTAGAATCCAATTACTAGAAAGGTTGTT          2517
```